# EUROPÄISCHE PATENTSCHRIFT

(11) **EP 0 624 161 B1**
(45) Veröffentlichungstag und Bekanntmachung des Hinweises auf die Patenterteilung: **24.02.1999**
(21) Anmeldenummer: 92924697.3
(22) Anmeldetag: 09.12.1992
(51) Int. Cl.: C07H 21/00, C07H 19/04, C12Q 1/68

(54) **2'-DESOXY-ISOGUANOSINE, ISOSTERE ANALOGE UND ISOGUANOSINDERIVATE SOWIE DEREN ANWENDUNG**
2'-DESOXY-ISOGUANOSINES, ISOSTER ANALOGUES AND ISOGUANOSINE DERIVATES AND THEIR USE
2'-DESOXY-ISOGUANOSINES, ANALOGUES ISOSTERES ET DERIVES D'ISOGUANOSINE, AINSI QUE LEUR UTILISATION

(30) Priorität: 09.12.1991 DE 4140463
(43) Veröffentlichungstag der Anmeldung: 17.11.1994
(73) Patentinhaber: BOEHRINGER MANNHEIM GMBH, 68298 Mannheim (DE)
(72) Erfinder: SEELA, Frank, D-4500 Osnabrück (DE); KASIMIERCZUK, Zigmunt, 02-776 Warszawa (PL); MÜHLEGGER, Klaus, D-8121 Polling (DE); VON DER ELTZ, Herbert, D-8120 Weilheim (DE)
(74) Vertreter: Fouquet, Herbert, Dr.
(86) Internationale Anmeldenummer: EP9202843
(87) Internationale Veröffentlichungsnummer: WO9312130

(56) Entgegenhaltungen:
- EP-A- 0 219 838
- EP-A- 0 269 445
- EP-A- 0 286 028
- WO-A-90/03370
- WO-A-90/06312
- NUCLEIC ACIDS RESEARCH. Bd. 11, Nr. 3, 11. Februar 1983, ARLINGTON, VIRGINIA US Seiten 871 - 882 H.B.COTTAM ET AL. 'A Convenient Synthesis of 6-Amino-1-B-D-ribofuranosylpyrazolo(3,4 -d)pyrimidin-4-one and Related 4,6-Disubstituted Pyrazolopyrimidine Nucleosides.'
- HELVETICA CHIMICA ACTA. Bd. 74, Nr. 8, 11. Dezember 1991, BASEL CH Seiten 1742 - 1748 Z.KAZIMIERCZUK ET AL. '168. 2'-Deoxyguanosine and Base-Modified Analogues: Chemical and Photochemical Synthesis.'

## Beschreibung

Nucleoside sind als Baustein der Nucleinsäure in der belebten Natur weit verbreitet. Sie kommen als Ribonucleoside in den Ribonucleinsäuren (RNS), als Desoxyribonucleoside in den Desoxyribonucleinsäuren (DNS) vor.

Natürlich vorkommende Nucleoside sind in der Regel aus einem Zuckerteil (Ribose bzw. Desoxyribose) und einem aglykonischen heterozyklischen Teil aufgebaut. Diese sogenannten Nucleobasen sind in der Regel Adenin, Guanin, Cytosin und Thymin, bzw. Uracil.

Darüber hinaus wurden in Naturstoffen Nucleoside gefunden, die nicht Bestandteile von Nucleinsäuren sind, wie z.B. Isoguanosin oder 1-Methyl-isoguanosin. Sie besitzen häufig interessante pharmakologische Eigenschaften.

Aufgrund dessen wurde Isoguanosinverbindungen und deren Herstellung Interesse entgegengebracht. So beschrieben Kazimierczuk et al. (1973, Acta Biochimica Polonica **20**, 395-402) Isoguanin-ribo- und -2'desoxyribonukleoside sowie deren 5'-phosphate. Didesoxy-Isoguanosin wurde im Zusammenhang mit der Herstellung von Didesoxynukleosiden erwähnt, die über einen enzymatisch katalysierten Ribose-Transfer hergestellt werden (WO 90/06312). Der Einfluß der Nukleobase und der Zuckerstruktur auf die protonenkatalysierte Furanosid-Pyranosid-Isomerisation von Tubercidin und dessen 2'-Desoxyderivaten wurde untersucht (Seela et al., 1986, J. Chem. Soc. Perkin Trans. II 1986). Bekannt sind auch Pyrazolo[3,4-d]Pyrimidinverbindungen (WO 90/03370) sowie Desaza-purin-nukleosidderivate (EPA 0 286 028) sowie Verfahren zu deren Herstellung.

Bei der Untersuchung von Poly(isoG) zeigte sich, daß die Poly(isoG) thermisch stabilere Sekundärstrukturen ausbildet als das Poly(G)(1976, Eur. J. Biochem. **65**, 183-192).

Die Aufgabe der vorliegenden Erfindung bestand darin 2'-Desoxy-isoguanosine. isostere Derivate und Isoguanosinderivate sowie deren Phosphorverbindungen zur Verfügung zu stellen. Besondere Aufmerksamkeit wurde den oben genannten Verbindungen entgegengebracht, die zur Synthese von Oligodesoxynucleotiden oder DNA-Fragmenten auf chemischen oder enzymatischen Wege geeignet sind. Oligodesoxynucleotide oder DNA-Fragmente, welche die erfindungsgemäßen Verbindungen enthalten. sind in biologischen Systemen zur Hemmung der Expression viraler Gene geeignet.

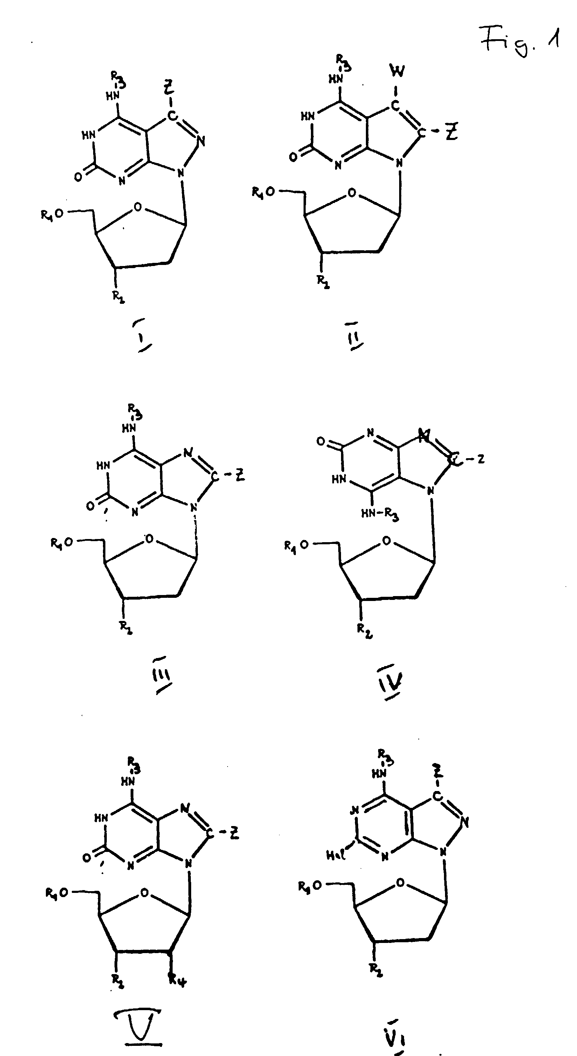

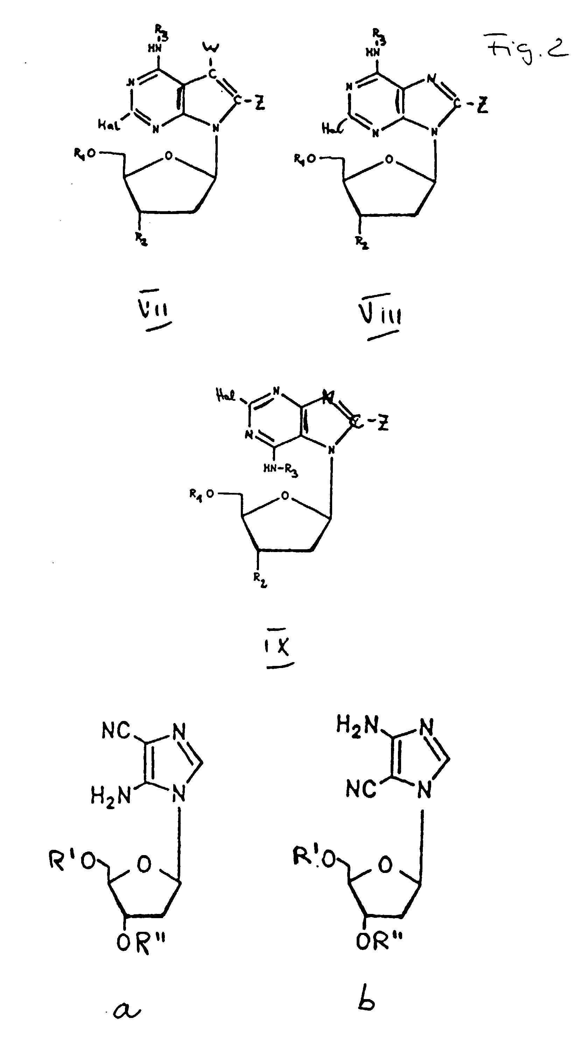

Gegenstand der Erfindung sind Verbindungen der allgemeinen Formeln II bis IV (Formelbilder siehe Fig. 1), worin
- R₁ =: Wasserstoff oder eine Schutzgruppe, PO₃H₂, P₂O₆H₃, P₃O₉H₄ oder die entsprechenden alpha-, beta- und gamma-Thiophosphate, mit der Maßgabe, daß für Formel III R₁ nicht P₃O₉H₄ bedeutet,
- R₂ =: Wasserstoff, Hydroxy, Phosphoramidit, Methylphosphonat, H-Phosphonat, eine Reporter- oder Interkalatorgruppe,
- R₃ =: Wasserstoff oder eine Schutzgruppe,
- W oder/und Z =: Wasserstoff, Halogen, NH-(CH₂)ₙNH₂ (n=2-12), R-CH₂COOH (R=Alkylen-C 1-8), eine Reporter- oder Interkalatorgruppe
bedeuten.

Die erfindungsgemäßen Verbindungen der Formeln II - IV werden hergestellt, indem man entweder Verbindungen der allgemeinen Formeln a oder b, worin
R' = R" ist und eine Acyl-Schutzgruppe wie z.B. p-Toluoyl- oder Benzoyl-darstellt, ausgeht, diese mit Aroylisocyanaten, z. B. Benzoylisocyanat umsetzt und das gewünschte Isoguanosin isoliert,
oder Verbindungen der allgemeinen Formeln VII bis IX, worin W, Z, R1, R2 und R3 die oben angegebene Bedeutung haben,
und Hal Chlor, Brom oder Jod darstellt
durch Bestrahlung photochemisch in die erfindungsgemäßen 2'-Desoxyisoguanosine umwandelt.

Ein besonders bevorzugtes Verfahren zur Herstellung der erfindungsgemäßen Verbindungen besteht darin, daß Desoxyguanosin bzw. Guanosin und deren Derivate mit Hexamethyldisilazan und Trimethylchlorsilazan persilyliert werden. Anschließend wird mit Ammoniak Tris(trimethyl)silyltriflat das 2,6-Diaminonucleosid hergestellt und selektiv in der 2-Position mit Nitrit zum Isoguanosin oder Desoxyguanosin desaminiert.

Die weitere Derivatisierung geschieht nach den dem Fachmann bekannten Methoden.

In den erfindungsgemäßen 2'-Desoxy-isoguanosinen kann der heterozyklische Teil vorzugsweise durch die entsprechenden Isostere 7-Desaza-isoguanin und ersetzt sein, wobei diese Basen zusätzlich noch Substituenten an C-7 des 7-Desaza- und an C-8 des Isoguanins tragen können. Solche Substituenten können z. B. Reportergruppen sein, wie unten beschrieben.
Besonders bevorzugt sind Wasserstoff, Halogen, NH-(CH₂)ₙNH₂, R-CH₂COOH, eine Reporter- oder Interkalatorgruppe.

Eine Diaminogruppe in der Position W und/oder Z kann eingeführt werden, indem eine Verbindung, in der W und/oder Z Wasserstoff ist, halogeniert wird (beispielsweise mit Bromwasser) und aus dem Bromid durch nukleophilen Austausch die Diaminogruppe eingeführt wird. Besonders bevorzugt wird eine Diaminopentyl- oder Diaminohexylgruppe eingeführt. Die gewünschte Verbindung kann aus dem Gemisch der Aminoverbindungen durch chromatographische Reinigung hergestellt werden.

Eine Carboxylfunktion kann eingesführt werden, indem die entsprechende halogenierte Verbindung mit Methyllithium umgesetzt und mit Halogen das Methylbromid hergestellt wird. Diese Verbindung wird mit Aminocarbonsäuren (z. B. Aminocapronsäure) zum Endprodukt umgesetzt.

Die Reporter- oder Interkalatorgruppen werden vorzugsweise in ihrer aktivierten Form (z. B. Hydroxysuccinimidester) an die Amino- oder Carboxylfunktion der erfindungsgemäßen Verbindungen gekoppelt.

Am 3'- und 5'-Ende des Zuckerteils können die erfindungsgemäßen Verbindungen alle dem Fachmann bekannten geeigneten Endgruppen enthalten. Bevorzugt sind für das 3'-Ende und für das 5'-Ende Wasserstoff, Mono-, Di- oder Triphosphat, eine Reportergruppe oder Interkalatorgruppe. Unter einer Reportergruppe im Sinne der Erfindung ist ein Hapten wie z.B. Biotin oder Digoxigenin oder ein Fluoreszenzfarbstoffrest zu verstehen. Geeignete Interkalatorgruppen sind bei Hélène, C. in "Antisense RNA and DNA, Curr. Commun. Mol. Biol.; Cold Spring Harbor Laboratory, Cold Spring Harbor, NY, 1987" beschrieben und sind vorzugsweise Phenanthrolin, Acridin, Aktinomycin bzw. dessen Chromophor oder Schwermetallkomplexbildner wie EDTA. Ebenso vorteilhaft sind solche Gruppen, die zur Vernetzung von Nucleinsäuren führen, wie z.B. Psoralen.

Aus den erfindungsgemäßen Verbindungen können durch Wasseraustritt zwischen 3'-OH und 5'-OH des Zuckerteils cyclische Phosphorsäurediester (3', 5'-Cyclophosphate) herstellt werden.

Die Herstellung der Phosphoamidite, H-Phosphonate und P-methylphosphoamidite von Isoguanosinen erfolgt analog der Herstellung der entsprechenden Desoxyisoguanosinderivate, wobei die 2'-OH-Funktion vorzugsweise über eine Triisopropylsilylgruppe geschützt wird.

Ein weiterer Gegenstand der Erfindung sind Basen- und Zucker-geschützte 2'-Desoxy-isoguanosine, -3'-H-phosphonate und -P-methyl-phosphoramidite. Diese Verbindungen sind als Nucleotidbausteine zur Herstellung von Oligonucleotiden geeignet.

Die erfindungsgemäßen Nucleotidbausteine enthalten vorzugsweise Schutzgruppen an den heterozyklischen Basen, sowie an den 5'-OH- und/oder 2'-OH-Funktionen der Ribose.

Als Schutzgruppe an den heterozyklischen Basen werden vorzugsweise Aminoschutzgruppen verwendet, wie z.B. Benzoyl-, Formamidin-, Isobutyryl- oder Diphenoxyacetyl-Gruppen.

Als 5'bzw. 2'-OH-Schutzgruppe des Zuckerteils werden vorzugsweise eine Triphenylmethyl-, Monomethoxytrityl-, Dimethoxytrityl-, t-Butyl-dimethylsilyl-, t-Butyldiphenylsilyl-, t-Butyl-methoxyphenylsilyl- oder die Pixyl-Gruppe verwendet.

Als Phosphoramidite sind 3'-O-(2-cyanoethyl)-N,N-diisopropyl-aminophosphane und die 3'-O-methyl-N,N-diisopropylamino-phosphane bevorzugt. Die H-Phosphonate werden vorzugsweise als Salze eingesetzt.

Die Herstellung der monomeren Nucleotidbausteine erfolgt nach den dem Fachmann geläufigen Methoden, beispielsweise wie sie in Gait, M.J. in "Oligonucleotide Synthesis, A Practical Approach", IRL Press, Ltd. (1984) beschrieben sind.

Die erfindungsgemäßen Verbindungen nach den allgemeinen Formeln II-IV und VII-IX können in Form ihrer jeweiligen 5'-Triphosphate bzw. α, β oder γ-Thiotriphosphate von DNA-Polymerasen in Oligonucleotide oder neusynthetisierte DNA eingebaut werden.

In einer besonders bevorzugten Ausführungsform sind diese erfindungsgemäßen Nucleotidbausteine ³²P- oder ³⁵S-markiert.

Ein weiterer Gegenstand der Erfindung ist daher ein Verfahren zur Herstellung von Oligo- und Polynucleotiden, die die erfindungsgemäßen Verbindungen als Bausteine enthalten. Solche Verfahren können sowohl chemischer als auch enzymatischer Natur sein.

Die chemische Synthese von Oligonucleotiden erfolgt nach den dem Fachmann geläufigen Methoden, beispielsweise wie sie in Gait, M.J., loc. cit. oder in Narang, S.A., "Synthesis and Application of DNA and RNA", Academic Press, 1987, beschrieben sind.

Die Herstellung der erfindungsgemäßen Oligonucleotide erfolgt in an sich bekannter Weise, beispielsweise nach der Phosphat-triester-, der Phosphittriester- oder H-phosphonat-Methode in homogener Phase oder an einem Träger. Bevorzugt werden die beiden letzteren Verfahren eingesetzt, wobei die Synthese üblicherweise mit automatisch arbeitenden Synthesegeräten erfolgt. Das Trägermaterial besteht dabei aus dem Fachmann bekannten anorganischen (controlled pore glass, Fractosil) oder organisch-polymeren Materialien (z.B. Polystyrol).

Ein weiterer Gegenstand der Erfindung ist deshalb ein Verfahren zur Herstellung von Oligonucleotiden, in denen je nach Sequenzdesign entsprechende erfindungsgemäße 2'-Desoxy-isoguanosine oder Isoguanosine eingesetzt werden und die aus 6 bis 100 Nucleotidbausteinen bestehen, nach dem Verfahren der Oligonucleotidsynthese, wobei ein Startnucleosid an einen festen Träger gebunden wird und anschließend durch schrittweise Kupplung mit entsprechend aktivierten monomeren Nucleotidbausteinen das gewünschte Oligonucleotid auf- gebaut wird, gegebenenfalls während und nach der Synthese dreiwertiger Phosphor zu fünfwertigem Phosphor oxidiert wird, das Oligonucleotid vom Träger mit einer ersten Base, heterocyclische Schutzgruppen mit einer zweiten Base, die 5'-Schutzgruppe mit einer Säure abgespalten und das Oligo nucleotid ggf. aufgereinigt wird. Die Auf reinigung geschieht vorzugsweise durch Reversed Phase- oder Anionenaustausch-HPLC. Danach schließt sich in der Regel eine Entsalzung, beispielsweise durch Dialyse an.

Weiterhin können die erfindungsgemäßen Oligonucleotide auch enzymatisch, unter Verwendung von Polymerasen, hergestellt werden. Solche Verfahren sind dem Fachmann unter den Begriffen "in vitro Transkription", "nick translation" [Rigby et al., J. Mol. Biol. 113, 237 (1977)] und "random priming" [Feinberg, A.P. und Vogelstein, B., Anal. Biochem. 137, 266 (1984)] bekannt.

Dabei werden prinzipiell 2'-Desoxynucleosid-5'-triphosphate oder Isoguanosin-5'-triphosphate von Polymerasen auf der Basis einer einzelsträngigen Template-Nucleinsäure mit Hilfe eines Startermoleküls (Primer/Promotor) in einen neusynthetisierten, dem ersten Strang basenkomplementären, zweiten Strang eingebaut. Durch die Verwendung der erfindungsgemäßen Nucleosid-5'-triphosphate und ihrer entsprechend substituierten Derivate können auf diese Weise z. B. geeignete Signal- oder Reportergruppen, wie Haptene oder Fluorophore in Nucleinsäuren eingebaut werden. Solche Techniken werden heute z. B. in Form der nichtradioaktiven Markierung von Biomolekülen breit angewendet.

Ein weiterer Gegenstand der Erfindung ist die Verwendung von Oligonucleotiden, in denen Guanosin bzw. Desoxyguanosin Bausteine ganz oder teilweise durch die erfindungsgemäßen Desoxy-isoguanosin oder Isoguanosin Bausteine ersetzt sind und die aus 6 bis 100 Nucleotidbausteinen bestehen, zur Herstellung eines Arzneimittels mit antiviraler Wirksamkeit.

2'-Desoxyisoguanosin-haltige Oligonucleotide bilden sowohl mit sich selbst als auch mit anderen herkömmlichen und modifizierten Oligonucleotiden Duplex- und Triplexstrukturen sowie auch Aggregate aus. Für 2'-Desoxyisoguanosin ergeben sich vielfältige Basenpaarungsmuster, die sich von denen von 2'-Desoxyguanosin unterscheiden und bei denen die Oligonucleotidstränge entweder parallel oder antiparallel angeordnet sein können.

### a) Aggregatstrukturen

Bei der Darstellung von 2'-Desoxyisoguanosin fällt auf, daß es wie 2'-Desoxyguanosin Gele bildet und extrem schwierig kristallisiert. Für guaninhaltige Oligonucleotide sind gelelektrophoretisch Aggregate nachgewiesen worden [66], die dem Strukturvorschlag von Zimmerman [67] folgen.

In dieser Struktur sind alle N⁷-Atome in einer Hoogsteen-Basenpaarung eingebunden. Die (iG_{d})₄-Struktur unterscheidet sich deutlich von der (G_{d})₄-Anordnung, da hier neben den O-Atomen zwei N⁷-Atome und zwei N³-Atome als Protonenakzeptoren fungieren. Deswegen kann sich beim 2'-Desoxy-isoguanosin eine Dimerstruktur bilden, bei der ein Molekül als 1H- und das andere als 3H-Tautomer vorliegt.

Die Wechselwirkung über N-7 ist bei 7-Desazapurinderivaten, wie 7-Desaza-2'-desoxyisoguanosin nicht möglich. Deshalb wird bei diesen Verbindungen keine Aggregatbildung beobachtet.

### b) Watson-Crick Duplex-Strukturen

Im Hinblick auf die Komplexierung von einzelsträngiger DNA oder RNA können iG_{d}-haltige Oligonucleotide Duplexstrukturen ausbilden. Hierbei sind parallele und antiparallele Anordnungen der Stränge möglich. Durch das veränderte Substituentenmuster an der Base und die möglichen parallelen Duplexstrukturen kann eine erhöhte Stabilität gegenüber Nucleasen erwartet werden.

### c) Triplexstrukturen von DNA-Duplexen mit iG_{d}-haltigen Oligonucleotiden

Oligonucleotide, die 2'-Desoxyisoguanosin als Monomerbausteine enthalten, können sowohl mit d(AT)- als auch mit d(GC)-Duplexen Triplexstrukturen ausbilden. In beiden Fällen können diese Strukturen im neutralen Medium entstehen; eine Protonierung ist nicht erforderlich. Damit kann die Komplexierung unter physiologischen Bedingungen erfolgen.

Die hier diskutierten Strukturen gelten prinzipiell für Oligoribo- und Oligodesoxyribonucleotide.

[66] J. Kim, Ch. Cheong und P.B. Moore, 'Tetramerization of an RNA oligonucleotide containing a GGGG sequence', Nature 1991, 351, 331.
[67] S.B. Zimmerman, 'X-ray Study by Fiber Diffraction Methods of a Self-aggregate of Guanosine-5'-Phosphate with the Same Helical Parameters as Poly (rG)', J. Mol. Biol. 1976, 106, 663.

Die erfindungsgemäßen Oligonucleotide und ihre Salze können als Medikament verwendet werden, z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann. Sie können auch rektal z.B. in Form von Suppositorien oder parenteral, z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg, Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole.

Die pharmazeutischen Präparate können auch Konservierungsmittel, Lösungsmittel Stabilisierungsmittel, Netzmittel, Emulgiermittel, Süßstoffe, Farbstoffe, Geschmacksmittel, Salze zur Veränderung des osmotischen Drucks, Puffer, Überzugsmittel oder Antioxidantien sowie ggf. andere therapeutische Wirkstoffe enthalten.

Die folgenden Beispiele erläutern die Erfindung näher:
(Der im weiteren verwendete Begriff "XAD-4" steht für "XAD-4"™ Serdolit (Fa. Serva))

### Beispiel 1

### 2'-Desoxy-isoguanosin [9-(2'-Desoxy-β-D-erythro-pentofuranosyl)-9H-isoguanin

**Methode A**: Zu einer Lösung von 400 mg (0,87 mmol) 5-Amino-1-[2'-desoxy-3',5'-di-O-(p-toluoyl)-β-D-erythro-pentofuranosyl)-4-imidazocarbonitril in 15 ml trockenem Acetonitril werden 0,6 ml (4,3 mmol) Benzoylisocyanat beiRaumtemperatur unter Rühren zugefügt. Innerhalb von 15 Min. bildet sich ein Niederschlag. Die Reaktionsmischung wird über Nacht gerührt und danach 30 Min. lang zum Rückfluß erhitzt. Das Lösungsmittel wird abdestilliert, der Rückstand in 100 ml einer Mischung von Isopropanol/25 % wässriger Ammoniaklösung (1:1) gelöst und die Lösung 2 Tage bei RT gerührt. Nach Eindampfen wird der Rückstand unter Rühren 2 x mit je 50 ml Ether extrahiert. Der Rückstand wird in 100 ml Wasser, welches mit einigen Tropfen 25 %iger Ammoniaklösung versetzt wurde, aufgenommen und filtriert. Das Filtrat wird auf etwa 30 ml Volumen eingeengt und auf eine XAD-4 - Säule (20 x 2 cm)aufgegeben. Durch Elution mit 200 ml Wasser werden die anorganischen Salze entfernt, die Elution mit Wasser/Isopropanol (9:1) liefert das gewünschte Produkt. Nach Eindampfen wird der Rückstand aus Ethanol kristallisiert.
Ausbeute: 100 mg = 43 % d. Th.
Farblose Kristalle, die Über 230 °C unter Zersetzung schmelzen.
DC (Cellulose, Fließmittel wassergesätt. n-Butanol): R_{f} = 0,25
UV (pH 1): λₘₐₓ 235 nm (5200), 284 (11500); (pH 7): 247 (9100), 292 (10100) (pH 13): 249 (6600), 284 (9800).
¹H-NMR (d₆DMSO): 2,18, 2,30 (2m, 2H, C-2'); 3,55 (m, 2H, C-5'); 3,84 (m, 1H, C-4');4,35 (m, 1H, C-3'); 5,30 (bs, 2H, OH-C-3' und OH-C-5'); 6,11 (t, J = 6,4 Hz, 1H, C-1'); 7,95 (s, 1H, C-8); 8,1 (bs, NH₂).
Elementaranalyse C₁₀H₁₃N₅O₄: Ber. C 44,94, H 4,90, N 26,21 ; gef. C 44,70, H 5,03, N 25,99.

**Methode B**: Eine Lösung von 200 mg (0,61 mmol) 2-Brom-2'-desoxy-adenosin in 250 ml Wasser wird in einem Quarz-Reaktor (bestückt mit einer 30 W Entkeimungslampe Philips/Holland; die Strahlung wird durch eine 2 mm Schicht von 20 %iger Essigsäure geleitet, um Licht unterhalb 230 nm auszufiltern) 30 Min. lang bestrahlt. Dann bringt man den pH mit Ammoniaklösung auf 8,0, konzentriert auf ein Volumen von ca. 20 ml und gibt auf eine XAD-4 - Säule (21 x 3 cm). Die Elution geschieht mit einem Gradienten Wasser auf 50 % Methanol, wobei das gewünschte Produkt bei 15-20 % Methanol eluiert wird. Die gesammelte Hauptfraktion wird eingedampft, der Rückstand aus Ethanol kristallisiert.
Ausbeute: 85 mg = 53 % d. Th.
Das Material ist chromatographisch und in seinen spektralen Daten identisch mit dem nach Methode A erhaltenen.

In analoger Weise wird 2'-Desoxy-isoguanosin aus 2-Chlor-2'-desoxy-adenosin nach einer Bestrahlungszeit von 1 Stunde in 52 %iger Ausbeute erhalten.

### Beispiel 2

### 7-(2'-Desoxy-β-D-erythro-pentofuranosyl)-7H-isoguanin

Zu einer Lösung von 300 mg (0.65 mMol) 4-Amino-1-(2'-desoxy-3',5'-di-O-(4-toluoyl)-β-D-erythro-pentofuranosyl)-1H-imidazol-5-carbonitril in 15 ml absolutem Acetonitril werden 0.27 ml (1.95 mMol) Benzoylisocyanat bei RT unter Rühren hinzugefügt. Nach 1 Stunde bildet sich ein weißer Niederschlag; die Suspension wird über Nacht gerührt, zur Trockene eingedampft und mit Isopropanol/25% wässriger Ammoniaklösung (75 ml, 1:1) versetzt. Nach 2-tägigem Rühren bei RT wird das Lösungsmittel abgedampft, der Rückstand in 75 ml Diethylether/Ethylacetat (1:1) aufgenommen und 1 Stunde bei RT gerührt. Die überstehende Lösung wird abdekantiert und der Rückstand mit 75 ml Wasser sowie 2 Tropfen 25%-iger wässriger Ammoniaklösung versetzt. Nach kurzem Rühren wird filtriert, das Filtrat auf 40 ml konzentriert und auf eine Amberlite XAD-4-Säule (25x3 cm) gegeben. Nach Waschen mit 200 ml Wasser eluiert man das Hauptprodukt mit 10%igem wässrigem Isopropanol und dampft anschließend das Lösungsmittel ab. Man kristallisiert aus Ethanol.
Ausbeute: 110 mg = 63 % d.Th.
Farblose Kristalle, die oberhalb 230°C unter Zersetzung schmelzen.
DC (Cellulose, Fließmittel wassergesättigtes n-Butanol): R_{f} = 0.15
UV (pH 1): λₘₐₓ 290 (8600); (pH 7): 282 (7100), 242 (7000); (pH 13): 285 (5700).
¹H-NMR (d₆DMSO): 2.25, 2.40 (2m, 2H, C-2'); 3.55 (m, 1H, C-5'); 3.84 (m, 1H, C-4'); 5.35 (bs, 3'-OH, 5'-OH); 6.12 (pt, J - 6.6 Hz, 1H, C-1'); 7.27 (bs, 2H, NH₂); 8.19 (s, 1H, C-8).
Elementaranalyse C₁₀H₁₃N₅O₄: Ber. C 44.94, H 4.90, N 26.2; gef. C 44.78, H 5.05, N 25.82.

### Beispiel 3

### 7-Desaza-2'-desoxy-isoguanosin-5'-triphosphat [4-amino-7-(2'-desoxy-β-D-erythro-pentofuranosyl)-3H,7H-pyrrolo[2,3-d]-pyrimidin-2-on-5'-triphosphat]

### 1. Stufe: 7-Desaza-2'-desoxy-isoguanosin

Eine Lösung von 85 mg (0,3 mmol) 4-Amino-2-chlor-7-(2'-desoxy-β-D-erythro-pentofuranosyl)-7H-pyrrolo[2,3-d]pyrimidin werden in 200 ml Wasser, welches 1 ml konzentrierte wässrige Ammoniaklösung enthält, gelöst und die Lösung 1 Stunde lang in einem Quarzreaktor bestrahlt, wie unter Beispiel 1, Methode B, beschrieben. Die Lösung wird anschließend auf 30 ml konzentriert und auf eine XAD-4-Säule (20x2 cm) gegeben. Das Harz wird anschließend mit 150 ml Wasser gewaschen, danach mit ca. 1 1 eines Gemisches aus Wasser/Isopropanol 9:1 eluiert. Die das Nucleosid enthaltenden Fraktionen werden gesammelt und eingedampft. Der Rückstand wird aus Ethanol kristallisiert.
Ausbeute: 46 mg = 58 % d.Th.
Farblose Nadeln vom Smp. 230 °C
Elementaranalyse C₁₁H₁₄N₄O₄: Ber. C 49,62, H 5,30, N 21,04; gef. C 49,7, H 5,4, N 21,1.

### 2. Stufe: 7-Desaza-2'-desoxy-isoguanosin-5'-triphosphat

Das Nucleosid aus Stufe 1 wurde nach Yoshikawa et al. [Tetrah. Lett. 50, 5065 (1967)] in das 5'-Monophosphat überführt; daraus wurde entsprechend dem Verfahren nach Hoard und Ott [J. Am. Chem. Soc. 87, 1785 (1965)] das gewünschte Triphosphat erhalten.
³¹P-NMR (0,1 m-EDTA/D₂O/Eth₃N): -8,20 (d, P-g), -10,5 (d, P-a), -21,2 (t,P-β)

### Beispiel 6

### 9-(2'-desoxy-β-D-erythro-pentofuranosyl)-6-{[1-(dimethylamino)methyliden]-9H-isoguanin (5)

200 mg (0,74 mmol) 2'-Desoxy-isoguanosin aus Beispiel 1 werden in 10 ml absolutem Dimethylformamid gelöst und mit 1,3 ml (7,6 mmol) N,N-Dimethylformamid-iethylacetal versetzt. Man rührt 24 h bei Raumtemperatur, engt im Vakuum ein und dampft den Rückstand mehrfach mit Toluol und Aceton ab. Anschließend wird an Kieselgel 60 H chromatographiert (Säule 25 x 4,5 cm, Elutionsmittel Chloroform/Methanol 8:2). Die Hauptfraktionen werden vereinigt, das Lösungsmittel abgedampft und der Rückstand aus Methanol umkristallisiert.
Ausbeute: 150 mg = 62 % d.Th.

Schwach gelbe Kristalle vom Smp. 178 °C (Z.).
DC (Kieselgel, Chloroform/Methanol 8:2): R_{f} = 0,62
UV (Methanol: λₘₐₓ = 338 (9400), 259 (6100), 223 (9100).
¹H-NMR (d₆DMSO): 2,20, 2,64 (2m, 2H, C-2'); 3,12, 3,22 (2s, 4H, (CH₃)₂N); 3,50 (2m, 2H, C-5'); 3,86 (m, 1H, C-4'); 4,38 (m, 1H, C-3'); 5,10 (t, 1H, OH-C-5'); 5,31 (d, 1H, OH-C-3'); 6,15 (pt, J = 6,25 Hz, 1H, C-1'); 8,08 (s, 1H, C-8); 9,2 (s, 1H, -N=CH).
Elementaranalyse: C₁₃H₁₈N₆O₄: Ber. C 48,44, H 5,63, N 26,07; gef. C 48,25, H 5,69, N 25,87.

### Beispiel 7

### 7-(2'-desoxy-β-D-erythro-pentofuranosyl)-6-[1-(dimethylamino)methyliden]-7H-isoguanin

300 mg (1,12 mmol) 7-(2'-Desoxy-β-D-erythro-pentofuranosyl)-7H-isoguanin werden in 10 ml absolutem Dimethylformamid gelöst und mit 2 ml (11,7 mmol) N,N-Dimethylformamiddiethylacetal versetzt und bei Raumtemperatur 24 h gerührt. Die Aufarbeitung geschieht wie unter Beispiel 6 beschrieben.
Ausbeute: 62 mg = 72 % d.Th.
Schwachgelbe Kristalle vom Smp. 208-209 °C.
DC (Kieselgel, Chloroform/Methanol 8:2): R_{f} = 0,56
UV (Methanol) λₘₐₓ = 326 (7800), 296 (6700), 245 (3200).
¹H-NMR (d₆DMSO): 2,30, 2,43 (2m, 2H, C-2'); 3,14, 3,23 (2s, 4H, (CH₃)₂N); 3,49 (2m, 2H, C-5'); 3,9 (m, 1H, C-4'); 4,30 (m, 1H, C-3'); 5,10 (t, 1H, OH- C-5'); 5,31 (d, 1H, OH-C-3'); 6,84 (pt, J = 6,25 Hz, 1H, C-1'); 8,32 (s, 1H, C-8); 8,8 (s, 1H, -N=CH).
Elementaranalyse: C₁₃H₁₈N₆O₄: Ber. C 48,44, H 5,63, N 26,07; gef. C 48,29, H 5,73, N 25,91

### Beispiel 8

### 9-(2-Desoxy-β-D-erythro-pentofuranosyl)-6-[1-(dimethylamino)methyliden]-2-chlor-adenin

300 mg (1,05 mmol) 2-Chlor-2'-desoxy-adenin [hergestellt nach Z. Kazimierczuk et al., J. Am. Chem. Soc. 106, 6379 (1984) )] werden in 3 ml wasserfreiem DMF gelöst und mit 0,9 ml (5,2 mmol) N,N-Dimethylformamid-diethylacetal versetzt. Man rührt 1 h bei Raumtemperatur, engt ein und dampft den Rückstand mehrfach mit Toluol ab. Anschließend wird an Kieselgel 60 H chromatographiert (Säule 15 x 5 cm, Elutionsmittel Dichlormethan/Methanol 9:1).
Ausbeute: 295 mg = 82,4 % d.Th.
Farbloses, schaumiges Produkt.

In analoger Weise kann nach Z. Kazimierczuk das 7-regioisomere hergestellt werden.

### Beispiel 9

### 9-(2'-Desoxy-β-D-erythro-pentofuranosyl)-5'-O-(4,4-dimethoxytriphenyl-methyl)-6-[1-(dimethylamino)methyliden]-2-chlor-adenin

340 mg (1 mmol) der Verbindung aus Beispiel 8 werden durch mehrmaliges Abdampfen mit wasserfreiem Pyridin getrocknet und anschließend in 2 ml Pyridin gelöst. Nach Zugabe von 440 mg (1,3 mmol) 4,4'-Dimethoxytriphenylmethylchlorid rührt man 1 Stunde bei Raumtemperatur. Daraufhin wird mit 30 ml einer 5 %igen wässrigen NaHCO₃-Lösung hydrolysiert und mit 3 x 50 ml Dichlormethan extrahiert. Die vereinigten organischen Phasen werden über Na₂SO₄ getrocknet, filtriert und das Lösungsmittel abgezogen. Nach Chromatographie an Kieselgel 60 H (Säule 20 x 5 cm, Laufmittel Dichlormethan/Methanol 9:1) und Eindampfen der Hauptfraktion wird das Produkt als amorphes Material erhalten.
Ausbeute: 390 mg = 60 % d.Th.
DC (Kieselgel, Fließmittel Dichlormethan/Methanol 9:1): R_{f} = 0,41
UV (Methanol): λₘₐₓ = 314 (27200), 236 (30500)
¹H-NMR (d₆DMSO): 2,37, 2,82 (2m, 2H, C-2'); 3,14, 3,22 (2s, 6H, (CH₃)₂N); 3,69, 3,71 (2s, 6H, 2 x O-CH₃); 3,98 (m, 1H, C-4'); 4,46 (m, 1H, C-3'); 5,39 (d, J = 4,5 Hz, 1H, OH-C-3'); 6,35 (pt, J = 6,2 Hz, 1H, C-1'); 6,72 - 7,3 (m, 13H, arom.); 8,37 (s, 1H, C-8); 8,84 (s, 1H, -N=CH).
Elementaranalyse C₃₄H₃₅N₆O₅Cl: Ber. C 63,49, H 5,49, N 13,06; gef. C 63,30, H 5,68, N 12,88.

### Beispiel 10

### 9-(2'-Desoxy-β-D-erythro-pentofuranosyl)-3'-(H-phosphonato)-5'-O-(4,4'-di-methoxytriphenylmethyl)-6-[1-(dimethylamino)methyliden]-2-chlor-adenin

Unter Argon-Atmosphäre werden zu einer Lösung von 12 ml absol. CH₂Cl₂, 135 ul (1,55 mmol) PCl₃ und 1,7 ml (15,2 mmol) N-Methyl-morpholin, bei Raum- temperatur 358 mg (5 mmol) 1,2,4-Triazol gegeben. Nach 30 Min. Rühren wird die Reaktionslösung auf 0°C abgekühlt und innerhalb von 10 Min. mit einer Lösung von 200 mg (0,31 mmol) der Verbindung aus Beispiel 9 in 10 ml CH₂Cl₂ versetzt. Man lässt weitere 20 Min. bei RT rühren und fügt anschließend 17 ml 1m-Triethylammoniumhydrogencarbonat-Puffer (TBK) (pH 7,7) zu. Die wässrige Phase wird 3 x mit je 15 ml CH₂Cl₂ extrahiert, die vereinigten organischen Phasen über Na₂SO₄ getrocknet und das Dichlormethan abgedampft. Nach Chromatographie an Kieselgel 60 H (Säule 20 x 5 cm, Elution zu Beginn mit 1 l Dichlormethan/Triethylamin 98:2, dann Dichlormethan/Methanol/Triethylamin 88:10:2) werden die Hauptfraktionen vereinigt, das Lösungsmittel abgezogen, der Rückstand in 20 ml CH₂Cl₂ aufgenommmen und mehrere Male mit 0,1 m TBK-Puffer ausgeschüttelt. Die vereinigten organischen Phasen werden über Na₂SO₄ getrocknet und eingeengt.
Ausbeute: 190 mg = 73 % d. Th.
DC (Kieselgel, Fließmittel Dichlormethan/Methanol/Triethylamin 88:10:2): R_{f} = 0,37
UV (Methanol): λₘₐₓ = 316 (19900), 235 (21200)
¹H-NMR (d₆DMSO): 0,9 - 1,2 (m, 9H, HN(CH2CH3)3); 2,92 (2m, 2H, C-2'); 2,60 - 2,64 (m, 6H, NH(CH2CH3)3); 3,14, 3,23 (2s, 6H, (CH3)2N9; 3,69, 3,71 (2s, 6H, 2 x OCH3); 4,16 (m, 1H, C-4'); 4,77 (m, 1H, C-3'); 6,63 (d, 1H, H-phosponat); 6,34 (pt, 1H, C-1'); 6,73 - 7,3 (m, 13H, arom.); 8,34 (s, 1H, C-8); 8,85 (s, 1H, -N=CH).
³¹P-NMR (d₆DMSO): 1,09 ppm (¹J (P-H) = 585,3 Hz, ³J (P-H) = 9,1 Hz). Elementaranalyse C₄₀H₅₁N₇O₇ClP: Ber. C 59,43, H 6,36, N 12,13 ; gef. C 59,26, H 6,50, N 11,93 .

### Beispiel 11

### 9-(2'-Desoxy-β-D-erythro-pentofuranosyl)-5'-O-(4,4'-dimethoxytriphenyl-methyl)-6-[1-dimethylamino)methyliden]-9H-isoguanin

322 mg (1 mmol) der Verbindung aus Beispiel 6 werden wie unter Beispiel 9 beschrieben, in das gewünschte 5'-Dimethoxytrityl-geschützte Nucleosid überführt.
Ausbeute: 355 mg = 57 % d. Th.
DC (Kieselgel, Dichlormethan/Methanol 9:1): R_{f} = 0,32
¹H-NMR (d₆DMSO): 2,35, 2,7 (2m, 2H, C-2'); 3,14, 3,24 (2s, 6H, (CH₃)₂N); 3,61, 3,69 (2s, 6H, 2xOCH₃); 4,10 (m, 1H, C-4'); 4,41 (m, 1H, C-3'); 5,28 (d, J = 4,5 Hz, 1H, OH-C-3'); 6.40 (pt, J = 6,2 Hz, 1H, C-1'); 6,68-7,2 (m, 13H, arom.); 8,4 (s, 1H, C-8); 8,8 (s, 1H, - N=CH).
Elementaranalyse C₃₄H₃₆N₆O₆: Ber. C 65,37, H 5,81, N 13,45; gef. C 65,23, H 5,92, N 13,18.

### Beispiel 12

### Träger(Fractosil)-gebundenes 9-[2'-Desoxy-β-D-erythro-pentofuranosyl-5'-O-(4,4'-dimethoxytriphenylmethyl)]-6-[(1-dimethyl-amino)methyliden]-9H-isoguanin

Zu einer Lösung von 312,3 mg (0,5 mmol) des vollständig geschütz-ten Nucleosides aus Beispiel 11 in 15 ml trockenem Pyridin werden 75 mg (0,6mmol) N,N-Dimethylamino-pyridin und 250 mg (2,5 mmol) Bernsteinsäureanhydrid gegeben und die Reaktionsmischung 72 Stdn. bei 40°C gerührt. Nach Zugabe von 5 ml Wasser wird zur Trockene eingedampft und der Rückstand mit 50 ml Toluol einrotiert. Man löst in 100 ml Dichlormethan und extrahiert nacheinander erst mit 30 ml 10 %iger wässriger Zitronensäurelösung, dann mit 30 ml Wasser. Nach Trocknen der organischen Phase über Na₂SO₄ wird das Lösungsmittel abdestilliert. Man erhält 335 mg (nahezu Theorie) des Succinates, welches ohne weitere Aufreinigung umgesetzt wird. Dazu wird dieses in 2,5 ml einer 5 %igen Lösung von Pyridin in Dioxan gelöst und mit 120 mg (0,9 mmol) p-Nitrophenol und 206 mg (1 mmol) Dicyclohexylcarbodiimid versetzt. Man rührt 3 Stunden bei RT, saugt vom ausgefallenen Dicyclohexylharnstoff ab und gibt zum Filtrat 3 ml DMF, 600 mg Fractosil ^{R} 200 (Merck, 450 ueq NH₂/g) und 0,625 ml Triethylamin. Es wird 4 Stunden bei RT geschüttelt, dann werden 0,2 ml Essigsäureanhydrid zugefügt und das Schütteln weitere 30 Min. fortgesetzt. Man saugt das Silicagel mit dem ge bundenen Nucleosid ab, wäscht nacheinander mit DMF, Ethanol und Ether und trocknet im Vakuum.

Die Beladung des Trägers mit Nucleosid wurde wie folgt bestimmt:
5 mg Fractosil-Träger wurden mit 10 ml 0,1 m p-Toluolsulfonsäurein Acetonitril behandelt. Nach ca. 10 min. Stehen wurde der Überstand bei 498 nm spektrophotometrisch ausgemessen, wobei eine Beladung von 35 umol Nucleosid pro Gramm Träger gefunden wurden (E_{DMT} = 70.000).

### Beispiel 13

### 9-(2'-Desoxy-β-D-erythro-pentofuranosyl)-5'-O-(4,4'-dimethoxy-triphenylmethyl)-6-[1-(dimethylamino)methylide]-9H-isoguanin-3'-(2-cyanoethyl)-N,N-diisopropylphosphoramidit

312,3 mg (0,5 mmol) des geschützten Nucleosides aus Beispiel 11 werden in 5 ml trockenem Tetrahydrofuran gelöst und dazu 0,275 ml (1,6 mmol) Ethyldiisopropylamin gegeben. Anschließend werden unter N² 0,125 ml (0,55 mmol) Chlor-β-cyanoethoxy-(N,N-diisopropylamino)- phosphan innerhalb von ca. 3 Minuten zugetropft. Man rührt das Reaktionsgemisch 30·Min. bei RT, fügt dann ca. 10 ml wässrige, 5 %ige NaHCO₃-Lösung zu und extrahiert daraufhin 2x mit je ca. 10 ml Dichlormethan. Die vereinigten organischen Phasen werden über Na₂SO₄ getrocknet, das Lösungsmittel abdestilliert und der Rückstand an Kieselgel 60 H mit dem Laufmittel CH₂Cl₂/Ethylacetat/Triethylamin, 45:45:10 chromatographiert.
Ausbeute: 380 mg = 94 % d. Th.
DC (Kieselgel, Fließmittel Dichlormethan/Ethylacetat/Triethylamin 45:45:10): R_{f} = 0,42 und 0,45 (2 Diastereomere).
³¹P-NMR (d₆DMSO): 147 und 152 ppm (2 Diastereomere)

### Beispiel 14

### Festphasensynthese des Oligonucleotides d(AAAA⁹ⁱG) via Phosphoramidit

Die Synthese des Oligonucleotides erfolgte im 1 umol-Maßstab unter Verwendung des kommerziell erhältlichen 5'-O-(4,4'-Dimethoxy-trityl)-N6-benzoyl-2'-desoxy-adenosin-3'-[(2-cyanoethoxy)-N,N-di-isopropylamino]phosphan und des Iso-G-Trägers aus Beispiel 12 nach dem Standardprotokoll des DNA-Synthesizers 380 B der Firma Applied Biosystems (User Manual). Die Abspaltung des 5'-tritylierten Oligomers vom Trägermaterial erfolgte bei Raumtemperatur in 25 % wässriger Ammoniaklösung.

Die Schutzgruppen der exocyclischen Aminofunktionen am Hetero-cyclus wurden durch 16-stündige Einwirkung von 25 %iger wässriger Ammoniak-Lösung bei 60°C entfernt. Die 4,4'-Dimethoxytrityl-Schutzgruppe des Oligomeren wurde durch Behandlung mit 80 %iger Essigsäure während 5 Min. bei RT abgespalten.

Die Reinigung des Oligonucleotides geschah mittels HPLC an einer RP-18-Säule mit folgendem Laufmittelsystem :
Laufmittel A: 5 % Acetonitril in 0,1 m Triethylammoniumacetat-Puffer, pH 7,0 Laufmittel B: 100 % Acetonitril
Gradient 25 Min. 0 - 20 % B in A

Die Entsalzung geschah an einer RP-18 - Silicagel-Säule, indem zuerst anorganisches Material durch Waschen mit Wasser entfernt, dann mit einem Gemisch Methanol/Wasser (3:2) das Oligomer eluiert wurde.
Ausbeute: 5,5 A₂₆₀-Einheiten = ca. 5o % d. Th.

### Beispiel 15

### Chemische Festphasensynthese des Oligonucleotides d(AAAA^{Cl}A) via Phosphonat

Die Synthese dieses Oligonucleotides mit einem 2-Chloradenosin-Baustein (^{Cl}A) geschah im Synthesemaßstab 1 umol unter Verwendung des kommerziell erhältlichen 5'-O-(4,4-Dimethoxytrityl)-N⁶-benzoyl-2'-desoxy-adenosin-3'-phosphonates und des Phosphonates aus Beispiel 10 nach Standardprotokoll (User Bulletin 47, 1988) im DNA-Synthesizer 380 B von Applied Biosystems.

Die Aufarbeitung und Reinigung des Oligomeren erfolgte wie unter Beispiel 13 beschrieben.
Ausbeute: 3 A₂₆₀-Einheiten = ca. 25 % d. Th.

### Beispiel 16

### Photochemische Umwandlung des Oligonucleotides aus Beispiel 15

2,0 A₂₆₀ - Einheiten des Oligomers aus Beispiel 14 wurden in 0,1 ml bidest. Wasser gelöst und mit einer 4 W Quecksilber-Resonanz-Lampe (Heraeus) mit einer Lichtintensität von 1,79x10¹⁷ Quanten x min⁻¹ x cm⁻² bestrahlt. Nach 45 Minuten war laut HPLC Befund eine nahezu quantitative umwandlung in das 2'-Desoxy-iso-guanosin enthaltende Oligomer erfolgt.

Das Produkt erwies sich bei Koinjektion mit dem Oligomer aus Beispiel 14 als identisch.

### Beispiel 17

### 2-Brom-9-(2'-deoxy-β-D-erythro-pentofuranosyl)-6-{[(dimethylamino)-methyliden]amino}-9H-purin

Die Verbindung wurde analog Beispiel 8 hergestellt mit der Ausnahme, daß folgende Mengen verwendet wurden: 2-Brom-2'-desoxyadenosin (330 mg, 1.0 mmol), DMF (3 ml) dimethylformamid diethyl acetal (0.85 ml, 5 mmol). Farbloser amorpher Feststoff (306 mg, 80%). TLC (CH₂Cl₂/MeOH, 9:1): R_{f} 0.33. UV (MeOH): 318 (28600), 238 (13000). 1H NMR ((D₆)DMSO): 2.32, 2.67 (2m, H-C(2')); 3.14, 3.23 (2s, CH3-N); 3.56 (m, H-C(5')); 3.86 (m, H-C(4')); 4.39 (m, H-C(3')); 4.95 (t, J = 5.5 Hz, HO-C(5')); 5.33 (d, J = 4.3 Hz, HO-C(3')); 6.31 ("t", J = 6.3 Hz, H-C(1')); 8.44 (s, H-C(8)); 8.85 (s, H-C(N=)). Anal. calc. for C₁₃H₁₇BrN₆O₃ (385.22): C 40.53, H 4.45, N 21.82; found: C 40.66, B 4.50, N 21.79.

### Beispiel 18

### 2-Brom-9-[2'-deoxy-5'-O-(4,4'-dimethoxytrityl)]-β-D-erythro-pentofuranosyl)-6-{[(dimethylamino)methyliden]amino}-9H-purin

Die Verbindung wurde wie in Beispiel 9 beschrieben hergestellt. Es wurden verwendet: Verbindung aus Beispiel 17 (250 mg, 0.65 mmol), pyridine (2 ml), 4,4'-dimethoxytrityl chlorid (330 mg, 0.98 mmol). FC vgl. 9. Farbloser Feststoff (260 mg, 58%). TLC (CH₂Cl₂/MeOH, 9:1): R_{f} 0.37. 1H NMR ((D₆)DMSO): 2.23, 2.68 (2m, H-C(2')); 3.01, 3.09 (2s, CH3-N); 3.57, 3.58 (2s, CH₃O); 3.84 (m, H-C(4')); 4.33 (m, H-C(3')); 5.24 (d, J = 4.5 Hz, HO-C(3')); 6.22 ("t", J = 5.8 Hz, H-C(1')); 6.6-7.2 (m, aromat. H); 8.21 (s, H-C(8)); 8.69 (s, H-C(N=)). Anal. calc. for C₃₄H₃₅BrN₆O₅ (687.59): C 59.39, H 5.13, N 12.22; found: C 59.46, H 5.40, N 12.09.

### Beispiel 19

### 9-[2'-Deoxy-5'-O-(4,4'-dimethoxytrityl)-β-D-erythropentofuranosyl] -6-[(dimethylamino)methyliden]-9H-isoguanin 3'-Triethylammonium Phosphonat.

Zu einer Lösung von PCl₃ (270 µl, 3.1 mmol) und N-methylmorpholin (3.5 ml) in CH₂Cl₂ (25 ml) wird 1,2,4-triazol (716 mg, 10 mmol) zugegeben. Nach 30 Minuten Rühren wird auf 0°C gekühlt und die Verbindung von Beispiel 11 (413 mg, 0.64 mmol) gelöst in CH₂Cl₂ (25 ml) langsam zugegeben. Nach 30minütigem Rühren bei Raumtemperatur wird die Mischung in 1 mol/l (Et₃NH)HCO₃ (35 ml) gegeben, geschüttelt und die Phasen getrennt. Die wäßrige Phase wird mit CH₂Cl₂ (3 x 30 ml) extrahiert. Die kombinierten organischen Extrakte werden getrocknet und der farblose Schaum an vier preparative Silica-Gel-Platten (20 x 20 cm) getrennt und entwickelt in CH₂Cl₂-MeOH-triethylamine 80:10:5. Der Rest aus der Hauptzone ergibt einen farblosen Schaum (320 mg, 63%). TLC (CH₂Cl₂/MeOH/Et₃N, 80:10:5): R_{f} 0.40. ¹H NMR (D₆)DMSO): ¹H NMR ((D₆)DMSO): 1.1-1.2 (m, CH₃CH₂-N); 2.25, 2.65 (2m, H-C(2')); 2.7-2.8 (m, CH₃CH₂N); 3.16, 3.25 (2s, CH₃-N); 3.25 (s, H-C(5')); 3.77, 3.78 (2s, CH₃O); 4.16 (m, H-C(4')); 4.79 (m, H-C(3')); 6.20 ("t", J = 6 Hz, HO-C(5')); 6.8-7.4 (m, aromat. H); 7.97 (s, H-C(8)); 9.15 (s, H-C(N=)). ³¹P NMR ((D₆)DMSO): 2.23 (¹J(PH) = 585 Hz, ³J(PH) = 8.1 Hz). Anal. calc. for C₄₀H₅₂N₇O₈P (789.87): C 60.82, H 6.63, N 12.41; found: C 60.80, H 6.79, N 12.41.

### Beispiel 20

### 2-Brom-9-[2'-deoxy-5'-O-(4,4'-dimethoxytrityl)]-β-D-erythro-pentofuranosyl)-6-{[(dimethylamino)methyliden]amino}-9H-purin 3'-Triethylammonium Phosphonat.

Aus der Verbindung nach Beispiel 18 wird analog Beispiel 19 das Phosphonat hergestellt. Farbloser Schaum (128 mg, 48%). TLC (CH₂Cl₂/MeOH/W(Et)₃, 88:10:2): R_{f} 0.35. ¹H NMR ((D₆)DMSO): 1.0-1.2 (m, CH₃CH₂-N); 2.23, 2.82 (2m, H-C(2')); 2.7-2.9 (m, CH₃CH₂N), 3.14, 3.23 (2s, CH₃-(N=)); 3.70, 3.71 (2s, CH₃O); 4.14 (m, H-C(4')), 4.72 (m, H-C(3')); 6.31 ("t", J - 6.2 Hz, H-C(1')); 6.63 (d, H-P, J = 585 Hz); 6.7-7.3 (m, aromat. H); 8.31 (s, H-C(8)); 8.82 (s, H-C(N=)).³¹P NMR((D₆)DMSO): 0.90 (1J(PH)=585 Hz, ³J(PH)=9.1 Hz).

### Beispiel 21

### 2-Chloro-9-(2'-deoxy-β-D-erythro-pentofuranosyl)-6-methylamino-9H -purin.

Die Suspension von 3',5'-bis-toluoyl-2,6-Dichlorpurinribosid (A) (1.08 g, 2 mmol) in CH₃N/MeOH (1:5, 30 ml) wird bei Raumtemperatur zwei Tage gerührt. Die Reaktionsmischung wird zur Trockenen im Vakuum eingedampft und der Rest an einer Silica-Gel-Säule chromatographiert (2 x 25 cm, CH₂Cl₂-MeOH (9:1)). Es werden farblose Nadeln aus Wasser erhalten (430 mg, 72%). M.p. 163-165°. TLC (CH₂Cl₂-MeOH, 9:1): R_{f} 0.35. UV (water): 270 (16800). ¹H-NMR ((D6)DMSO): 2.30, 2.65 (2m, H₂-C(2'), 2.92 (d, J = 4.1 Hz, CH3-N), 3.55 (m, H₂-C(5')), 3.86 (m, H-(C-4')), 4.39 (m, H-(C-3')), 4.96 (t, J = 5.3 Hz, OH-C(5')), 5.32 (d, J = 4.0 Hz, OH-C(3')), 6.27 ("t", J = 6.8 Hz, H-C(1')), 8.26 (d, J - 4.2 Hz, NH), 8.35 (s, H-C(8). Anal. calc. for C₁₁H₁₄ClN₅O₃ (299.7): C 44.08, H 4.71, N 23.37; found: C 43.96, H 4.90, N 23.24.

### Beispiel 22

### 2-Chlor-9-(2'-deoxy-β-D-erythro-pentofuranosyl)-6-hydroxyethyl-amino-9H-purin.

Wie für Beispiel 21 beschrieben, wird Hydroxyethylamin (915 mg, 15 mmol) mit (A) (810 mg, 1.5 mmol) in MeOH (25 ml) umgesetzt. Farblose Nadeln (310 mg, 63%) aus EtOAc/aceton. M.p. 167-168°. TLC (CH₂Cl₂-MeOH, 9:1): R_{f} 0.15. UV (water): 271 (18700)· ¹H-NMR ((D₆)DMSO): 2.30 and 2.65 (2m, H-C(2')), 3.56 (m, H-C(5'), H-CH₂(N)), 3.86 (q, H-C(4')), 4.39 (bs, H-C(3')), 4.76 (t, J - 4.6 Hz, OH-C(ethyl)), 4.95 (t, J = 5.5 Hz, OH-C(5')), 5.30 (d, J = 4.1 Hz, OH-C(3')), 6.27 (t, J = 6.7 Hz, H-C(1')), 8.13 (bs, H-N(6)), 8.35 (s, H-C(8)). Anal. calc. for C₁₂H₁₆ClN₅O₄ (329.7): C 43.71, H 4.89, N 21.24; found: C 43.59, H 4.80, N 21.09.

### Beispiel 23

### 2-Chlor-6-butylamino-9-(2'-deoxy-β-D-erythro-pentofuranosyl)-9H-purin.

Wie in Beispiel 21 beschrieben, wird Verbindung (A) (540 mg, 1 mmol) in MeOH (15 ml) mit N-butylamin (730 mg, 10 mmol) bei 37°C umgesetzt. Die Reinigung erfolgt an 20 x 20 cm präparativen Silica-Gel-Platten mit CH2Cl₂-MeOH (9:1) als Laufmittel. Es wird ein farbloser Feststoff erhalten (210 mg, 61%) der als Nadeln kristallisiert (Wasser). M.p. 159°. TLC (CH2Cl2-MeOH, 9:1): Rf 0.50. UV (water): 271 (18800). ¹H-NMR ((D6)DMSO): 0.88, 1.31, 1.56, 3.40 (m, aliphat. H), 2.27 and 2.63 ( 2m, H-C(2')), 3.56 (m, H-C(5')), 3.86 (bs, H-C(4')), 4.40 (bs, H-C(3')), 4.95 (t, J = 5.3 Hz, OH-C(5')), 5.30 (d, J = 3.8 Hz, OH-C(3')), 6.27 ("t", J = 6.7 Hz, H-C(1')), 8.30 (bs, H-N(6)), 8.34 (s, H-C(8)). Anal. calc. for C₁₄H₂₀ClN₅O₃ (341.8): C 49.20, H 5.90, N 24.49; found: C 49.16, H 5.85, N 24.30.

### Beispiel 24

### 2-Chlor-6-cyclohexylamino-9-(2'-deoxy-β-D-erythro-pentofuranosyl) -9H-purin.

Cyclohexylamin (1.0 g, 10 mmol) wird zu einer gerührten Lösung von Verbindung (A) (1.08 g, 2 mmol) in MeOH (30 ml) zugegeben. Es wird für zwei Tage bei Raumtemperatur weitergerührt. Die Reaktionsmischung wird mit Natriummethoxid (3 ml, 1 mol/l in MeOH) umgesetzt und ein weiterer Tag gerührt. Die Reaktionsmischung wird im Vakuum eingedampft (Öl) und chromatographisch an einer Silica-Gel-Säule (2.5 x 25 cm, with CH3Cl-MeOH (9:1) als Laufmittel) gereinigt. Die nukleosidhaltigen Fraktionen werden im Vakuum zur Trockenen eingedampft. Der Rest ergibt aus EtOAc farblose Kristalle. (460 mg, 63%). M.p. 160-162°. TLC (CH2C12-MeOH, 9:1): R_{f} 0.63. UV (water): 273 (19600). ¹H-NMR (D6(DMSO)): 1.0 - 2.0 (m, H - (cyclohexyl)), 2.25 and 2.60 (2m, H-C(2')), 3.55 (m, H-C(5')), 3.86 (q, H-C(4')), 4.38 (bs, H-C(3')), 4.97 (t, J = 5.6 Hz, OH-C(3')), 5.32 (d, J = 4.2 Hz, OH-C(5')), 6.26 ("t", J = 6.6 Hz, H-C(1')), 8.15 (d, J = 8.4 Hz, H-N(6)), 8.34 (s, H-C(8)). Anal. calc. for C₁₆H₂₂ClN₅O₃ (367.8): C 52.24, H 6.03, N 19.04; found: C 52.11, H 5.96, N 18.88.

### Beispiel 25

### 2-Chlor-6-benzylamino-(2'-deoxy-β-D-erythro-pentofuranosyl)-9H-purin.

Wie in Beispiel 24 beschrieben, wird Benzylamin (1.07 g, 10 mmol) mit Verbindung (A) (920 mg, 1.7 mmol) MeOH (30 ml) umgesetzt. Die Reinigung erfolgt an einer Silica-Gel-Säule (3 x 24 cm, EtOAc (200 ml) und EtOAc-MeOH (9:1) als Laufmittel) chromatographiert. Es werden farblose Kristalle aus EtOH/Wasser erhalten (390 mg, 61%) M.p. 147°. TLC (CH2Cl2-MeOH, 9:1): Rf = 0.60. UV (water): 273 (21 600). ¹H-NMR ((D₆)DMSO): 2.30 and 2.60 (2m, H-C(2')), 3.53 (m, H-C(5')), 3.85 (q, H-C(4')), 4.39 (bs, H-C(3')), 4.64 (d, J = 5.7 Hz, H-(CH₂)), 4.96 (t, J = 5.9 Hz, OH-C(5')), 5.33 (d, J = 4.2 Hz, OH-C(3')), 6.27 ("t", J = 6.6 Hz, H-C(1')), 7.25-7.32 H-C(phenyl)), 8.37 (s, H-C(8)), 8.89 (t, J = 4.8 Hz, H-N(6)). Anal. calc. for C₁₇H₁₈ClN₅O₃ (375.8): C 54.33, H 4.83, N 18.64; found: C 54.27, H 4.85, N 18.54.

### Beispiel 26

### 2-Chlor-9-(2'-deoxy-β-D-erythro-pentofuranosyl)-6-methoxy-9H-purin.

Eine Suspension von Verbindung (A) (810 mg, 1.5 mmol) in MeOH (30 ml) wird mit Natriummethoxid/MeOH (4.5 ml, 1 mol/l) behandelt und 3 Stunden bei Raumtemperatur gerührt. Nach 30 Minuten bildet sich eine klare Lösung. Die Reaktionsmischung wird eingedampft und der Rest an einer Silicasäule 60H (3 x 22 cm mit EtOAc (200 ml) und EtOAc-MeOH (9:1) als Laufmittel) eluiert. Die nukleosidhaltigen Fraktionen werden gesammelt, im Vakuum eingedampft und der Rest kristallisiert aus EtOAc. Es werden farblose Kristalle (290 mg, 64%) erhalten. M.p.141-142°. TLC (CH₂Cl₂-MeOH, 9:1): Rf = 0.54. UV (water): 258 (12300). ¹H-NMR ((D₆)DMSO): 2.35 and 2.70 (2m, H-C(2')), 3.56 (m, H-C(5')), 3.85 (q, H-C(4')), 4.10 (s, H-C(6-OCH₃)), 4.42 (bs, H-C(3')), 4.94 (t, J = 5.5 Hz, OH-C(5')), 5.36 (d, J = 4.3 Hz, OH-C(3')), 6.35 ("t", J = 6.6 Hz, H-C(1')), 8.60 (s, H-C(8)). Anal. calc. for C₁₁H₁₃ClN₄O (300.7): C 43.94, H 4.36, N 18.63; found: C 44.08, H 4.45, N 18.63.

### Beispiel 27

### 6-Amino-2-chlor-9-(2'-deoxy-3',5'-di-O-acetyl-β-D-erythro-pentofuranosyl)-9H-purin.

Die gerührte Suspension von 2-Chlor-2'-desoxyadenosin (850 mg, 3 mmol) in Pyridin (10 ml) wird mit Essigsäureanhydrid (10 ml) behandelt, wobei sich das Ausgangsmaterial innerhalb von 30 Minuten löst. Nach 3 Stunden wird die Mischung im Vakuum eingedampft (Öl), dreimal mit Toluol/EtOH aufgenommen und eingedammpft. Der ölige Rest wird im Hochvakuum getrocknet und aus EtOH kristallisiert. (1.03 g, 92%). M.p. 173-175°. TLC (CH₂Cl₂-MeOH, 95:5): Rf = 0.52. UV (MeOH/H₂O): 264 (15300). ¹H-NMR ((D₆)DMSO): 1.82 and 2.01 (2s, H-(CH₂CO)), 2.55 and 3.00 (2m, H-C(2')), 4.25 (m, H-C(4') and H-C(5')), 5.37 (m, H-C(3')), 6.29 ("t", J = 6.5 Hz, H-C(1')), 7.85 (s, NH₂), 8.37 (s, H-C(8)). Anal. calc. for C₁₄H₁₆ClN₅O₅ (369.8): C 45.48, H 4.06, N 18.94; found: C 45.61, H 4.12, N 18.90.

### Beispiel 28

### 6-Amino-8-brom-2-chlor-9-(2'-deoxy-3',5'-di-O-acetyl-β-D-erythro-pentofuranosyl)-9H-purin.

Eine Lösung von 3,5-Diacetyl-2-chlor-2-Desoxyadenosin (400 mg, 1.08 mmol) in dioxan (16 ml) und aq. Natrium acetat (pH 4.7, 0.5 M, 4 ml) wird gerührt und eine Lösung von Br₂ (240 mg, 1.5 mmol) in Dioxan innerhalb 15 Minuten zugegeben. Es wird für 15 Minuten weitergerührt (TLC-Kontrolle). Die Mischung wird mit CHCl₃ (50 ml) verdünnt und mit Wasser (50 ml), Natriumbicarbonat (50 ml, sat.), 1 % Na₂S₂O₄ (50 ml), und Wasser (2 x 50 ml). Die organische Phase wird über Na₂SO₄ getrocknet und zur Trockenen eingedampft. Der Rest wird aus EtOH kristallisiert. Es ergeben sich farblose Kristalle von (8). (370 mg, 76%). M.p. 163-164°. TLC (CH₂Cl₂-MeOH, 95:5): Rf = 0.65. UV (MeOH/H₂O, 1:1): 269 (17500). ¹B-NMR ((D₆)DMSO): 1.95 and 2.09 (2s, H-C(CH2CO), 2.55 and 3.45 (2m, H-C(2'), 4.17 (m, H-C(5'), 4.34 (m, H-C(4'), 5.33 (q, H-C(3'), 6.29 ("t", J = 6.8 Hz, H-C(1'), 7.96 (s, NH₂). Anal.
calc. for C₁₄H₁₅BrClN₅O₅ (448.7): C 37.48, H 3.37, N 15.61; found: C 37.63, H 3.43, N 15.66.

### Beispiel 29

### 6-Amino-8-brom-2-chlor-9-(2'-deozy-β-D-erythro-pentofuranosyl)- 9H-purin. (8-Brom-2-chlor-2'-deoxyadenosin.)

Zu einer Lösung der Verbindung aus Beispiel 28 (300 mg, 0.67 mmol) in in MeOH (10 ml) wird Ammoniak in Methanol (10 ml, gesättigt bei 0°C) zugegeben. Die Reaktionsmischung wird über Nacht bei 4°C gerührt. Es bilden sich hellgelbe chromatographische reine Kristalle (192 mg, 79 %). Eine analytische Probe wird aus Ethanol kristallisiert. 190° (decomp.). TLC (CH₂Cl₂-MeOH, 9:1): Rf = 0.57. UV (water): 269 (16300). ¹H-NNR ((D₆)DMSO): 2.20 and 3.15 (2m, H-C(2')), 3.45 and 3.62 (2m, H-C(5')), 3.82 (q, H-C(4')), 4.45 (bs, H-C(3')), 4.85 (t, J = 6.2 Hz, OH-C(5')), 5.35 (d, J - 4.2 Hz,OH-C(3')), 6.23 ("t", J = 7.1 Hz, H-C(1')), 7.99 (s, NH₂). Anal. calc. for C₁₀H₁₁BrClN₅O₃ (364.6): C 32.94, H 3.04, N 19.21; found: C 33.12, H 3.11, N 19.22.

### Beispiel 32

### 2-Amino-(2'-desoxy-β-D-erythropentofuranosyl)adenin

5.0 g (18.6 mmol) Desoxyguanosin werden mit 200 ml Hexamethyldisilazan (HMDS) und 0.5 ml Trimethylchlorsilan (TCS), wie die entsprechende Riboverbindung [2][3], 10 h bei 145°C silyliert. Anschließend wird der Überschuß an HMDS unter Normaldruck abdestilliert. Der zurückgebliebene trübe Sirup ([2]) wird in einem Gemisch von 30 ml abs. Toluol und 2 ml HMDS aufgenommen. Diese Mischung wird in einen 300 ml Autoklaven überführt. Nach Zusatz von 4 ml einer 0.5 M Lösung (2 mmol) von Tris(trimethyl)silyltriflat im abs. Toluol wird bei 0°C 0.5 h NH₃ (5 bar) aufgepreßt; dann wird bei 145°C (externe Temperaturregelung), 48 h erhitzt. Nach Abkühlen auf Raumtemperatur wird das NH₃ vorsichtig abgeblasen. Der feste Rückstand wird in 150 ml Methanol suspendiert (Ultraschallbad), mit 150 ml Wasser versetzt und 4 h bei 100°C transsilyliert. Nach Abdestillieren des Methanols werden 250 ml Wasser zugegeben, die Lösung mit Aktivkohle versetzt, heiß filtriert und mit 100 ml heißem Wasser nachgewaschen. Das gelbe Filtrat wird auf eine Dowex-lx2 Säule (20 x 2 cm, OH⁻) aufgetragen. Elution mit 500 ml Wasser liefert eine Hauptzone, aus der man nach dem Abdampfen das Produkt (1.2 g, 24.2 %) als gelbes Pulver erhält. Das Produkt ist identisch mit einer authentischen Probe [1][4-6]. DC(Kieselgel, CH₂Cl₂/MeOH 4:1): R_{f} = 0.4. UV (MeOH): = λₘₐₓ217 (23500), 256 (8900), 282 (9900). ¹³C-NMR in ((D6)DMSO): 160.2 C(6); 156.3 C(2); 151.3 C(4); 135.9 C(8); 113.5 C(5); 87.8 C(4'); 83.2 C(1'); 71.1 C (3'); 62.1 C(5').

### Beispiel 33

### 6-Amino-9-(2'-desxoxy-β-D-erythropentofuranosyl)-1,9-dihydro-2H-purin-2-on (2'-Desoxyisoquanosin).

Analog zum Ribonucleosid [7] wird eine Lösung von 300 mg (4.3 mmol) Natriumnitrit in 5 ml heißem H₂O wird bei 50°C mit 300 mg (1.1 mmol) der Verbindung aus Beispiel 32 versetzt und langsam insgesamt 0.45 ml (7.8 mmol) Eisessig zugetropft (Schäumenl). Nach 5 min wird mit 10 ml H₂O verdünnt und mit verd. Ammoniak auf pH 8 eingestellt und dann weitere 300 ml H₂O zugegeben. Man adsorbiert an Serdolit AD-4 Ionentauscher (Serva-Deutschland, 4 x 22 cm) und wäscht mit 500 ml H₂O. Eine Mischung von H₂O/i-PrOH (95:5) etwa 500 ml, eluiert eine Hauptzone, aus der nach Abdampfen die Verbindung als gelbliches Pulver (120 mg, 40.8 %) erhalten wird. DC (Kieselgel, mit H₂O gesättigtes n-Butanol): R_{f} 0.25. UV (MeOH): λₘₐₓ248 (8100), 297 (9900). ¹³C-NMR in ((D6)DMSO): 156.4 C(6); 137.7 C(8); 153.0 C(2); 109.8 C(5); 88.1 C(4'); 83.9 C(1'); 71.2 C(3'); 62.0 C(5')[8].

### Beispiel 35

### 6-Amino-2-chlor-7-(2'-deoxy-β-D-erythropentofuranosyl)purin

Eine Lösung von 2,6-dichlor-7-(2'-deoxy-3',5'-di(O-(p-toluoyl)-β-D-erythropentofuranosyl)-purin (600 mg, 1.11 mmol) in methanolischem Ammoniak (60 ml, gesättigt bei 0°C) wird bei 80°C für 24 Stunden gerührt. Die Mischung wird im Vakuum eingedampft und der Rest auf Silicagel 60H (Säule 15 x 4 cm) chromatographiert. Kristallisation aus MeOH/iso-PrOH ergibt farblose Kristalle (210 mg, 66.5 %) mit einem Schmelzpunkt >250°C. UV (MeOH): λₘₐₓ276 nm (E = 7200). ¹H NMR ([D₆]DMSO) δ = 2.30 und 2.40 (m, 2'-H). 3.56 (m, 5'-H), 3.92 (m, 4'-H), 4.40 (m, 3'-H), 5.18 (t, J = 5.0 Hz, 5'-OH), 5.42 (d, J = 4.5 Hz, 3'-OH), 6.31 (pt, J = 6.5 Hz, 1'-H), 7.48 (s, NH₂), 8.56 (s, 8-H).

| | | | | |
|---|---|---|---|---|
| C₁₀H₁₂N₅O₃Cl (285.67) | Calcd. | C 42.05 | H 4.23 | N 24.51 |
| | Found | C 42.08 | H 4.25 | N 24.58 |

### Beispiel 36

### 7-(2'-Deoxy-β-D-erythro-pentofuranosyl)-7H-isoquanin

Eine Lösung der Verbindung aus Beispiel 35 (44 mg, 0.16 mmol) in Wasser (75 ml) wird 1 ml wäßrige Ammoniaklösung zugegeben. Die Mischung wird acht Stunden mit UV-Licht in einem Quarzbehälter bestrahlt. Die Lösung wird in Vakuum eingedampft und der Rest in Wasser (50 ml) gelöst und auf einer XAD-4-Säule (3 x 20 cm) chromatographiert. Die Säule wird mit Wasser (500 ml) gewaschen und das Produkt mit Wasser/Isopropanol 1:1) eluiert. Die nukleotidenthaltenden Fraktionen werden gesammelt in Vakuum eingedampft und aus Ethanol kristallisiert.
[1] Sigma Chemie GmbH, 8024 Deisenhofen, Deutschland.
[2] H. Vorbrüggen, K. Krolikiewicz, Liebigs Ann. Chem. 1976, 745
[3] M. J. Robins, F. Hansske, S. E. Bernier, Can. J. Chem. 1981, 59
[4] R. H. Iwamoto, E.M. Acton, L. Goodman, J. Med. Chem. 1963, 6, 684.
[5] J. A. Montgomery, K. Hewson, J. Med. Chem. 1969, 12, 498.
[6] R. Fathi, B. Goswami, Pei-Pei Kung, B. L. Gaffney, R. A. Jones, Tetrahedron Lett. 1990, 31, 319.
[7] J. Davoll, J. Am. Chem. Soc. 1951, 73, 3174.
[8] Z. Kazimierczuk, R. Mertens, W. Kawozynski, F. Seela, Helv. Chim. Acta 1991, 74, 1742.

## Patentansprüche

1. Oligonukleotide, die eine oder mehrere der in den Verbindungen der allgemeinen Formeln II - IV genannten heterocyclischen Basen enthalten 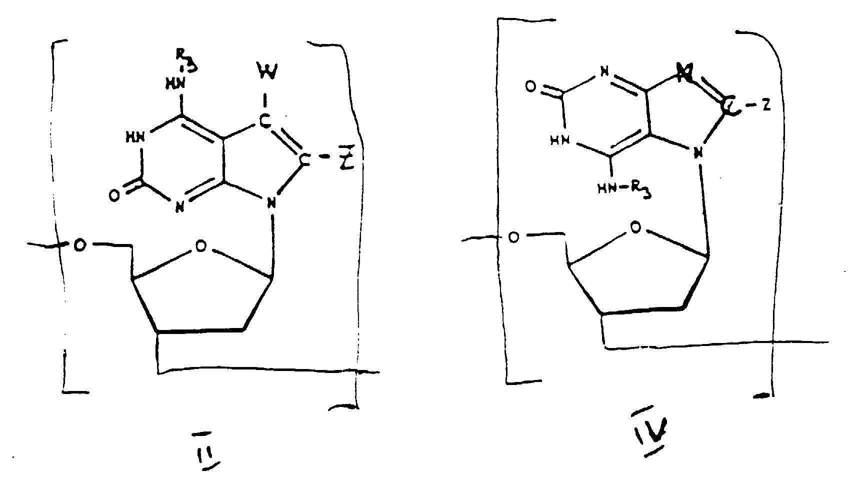 worin
R₃ = Wasserstoff oder eine Schutzgruppe,
W und Z können unabhängig voneinander bedeuten = Wasserstoff, Halogen, NH-(CH₂)nNH₂ (n = 2-12), R-CH₂COOH (R = Alkylen-C 1-8),
eine Reporter- oder Interkalatorgruppe bedeuten
und 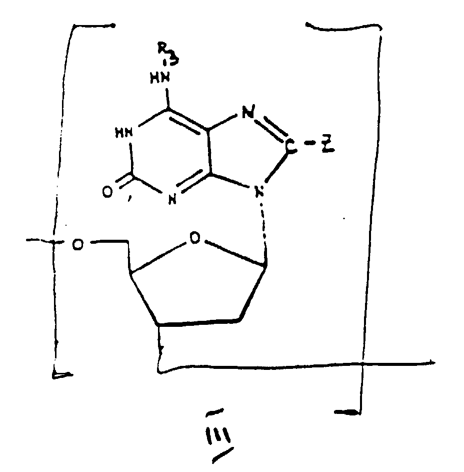 worin
R₃ = Wasserstoff oder eine Schutzgruppe,
Z bedeutet = Wasserstoff, Halogen, NH-(CH₂)ₙNH₂ (n = 2-12), R-CH2COOH (R = Alkylen-C 1-8),
eine Reporter- oder Interkalatorgruppe bedeuten
und die zur Ausbildung paralleler Nukleinsäurekomplexe befähigt sind.

2. Oligonukleotide nach Anspruch 1, die mit einem Isocytidin enthaltenden zweiten Oligonukleotid komplementäre Doppelstränge ausbilden können.

3. Oligonukleotide nach den Ansprüchen 1 und 2, deren Internukleotidbindung Methylphosphonat- und/oder Phosphothioatgruppen enthalten.

4. Oligonukleotide nach Ansprüchen 1 bis 3, dadurch gekennzeichnet, daß sie aus 6 bis 100 Nukleotidbausteinen bestehen.

5. Verbindungen der allgemeinen Formel II und IV, 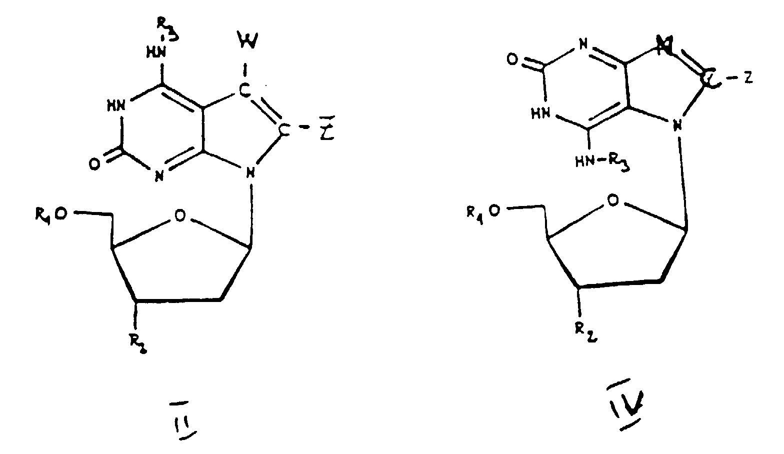 worin
R₁= Wasserstoff oder eine Schutzgruppe, PO₃H₂,P₂O₆H₃, P₃O₉H₄ oder die entsprechenden alpha-, beta- und gamma-Thiophosphate
R₂= Hydroxy, Phosphoramidit, Methylphosphonat, H-Phosphonat, eine Reporter- oder Interkalatorgruppe mit der Maßgabe, daß R₂ nicht OH ist, wenn R₁, R₃, W und Z jeweils Wasserstoff darstellen
R₃= Wasserstoff oder eine Schutzgruppe,
W und Z können unabhängig voneinander bedeuten = Wasserstoff, Halogen, NH-(CH₂)nNH₂ (n = 2-12), R-CH₂COOH (R = Alkylen-C 1-8),
eine Reporter- oder Interkalatorgruppe bedeuten.

6. Verbindungen der allgemeinen Formel III, 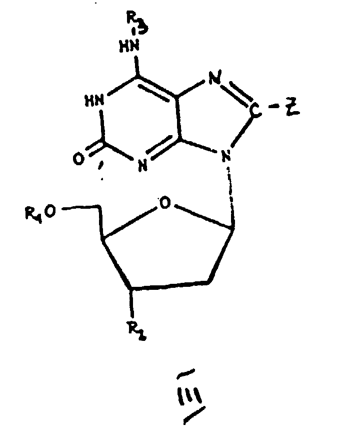 worin
R₁= eine Schutzgruppe
R₂= Phosphoramidit, H-Phosphonat, Methylphosphonat, eine Reporter- oder Interkalatorgruppe
R₃ = Wasserstoff oder eine Schutzgruppe,
W und Z können unabhängig voneinander bedeuten = Wasserstoff, Halogen, NH-(CH₂)ₙNH₂ (n = 2-12), R-CH₂COOH (R = Alkylen-C 1-8),
eine Reporter- oder Interkalatorgruppe
bedeuten.

7. Verbindungen nach den Ansprüchen 5 und 6, in denen die heterocyclischen Basen Schutzgruppen tragen.

8. Verfahren zur Herstellung der Oligonukleotide gemäß den Ansprüchen 1 - 4, dadurch gekennzeichnet, daß die Kondensation mit Verbindungen gemäß Anspruch 5 - 7 erfolgt.

9. Verfahren gemäß Anspruch 8, dadurch gekennzeichnet, daß bei den Verbindungen der allgemeinen Formeln II -IV R₁ ein Triphosphat oder die α-, beta- oder gamma-Thiophosphatanaloga dieser Phosphorsäureester darstellt und R₂ eine Reportergruppe oder eine Interkalatorgruppe darstellt und die Kondensation durch Polymerasen enzymatisch nach den Verfahren der "nick translation" und des "random priming" erfolgt.

10. Verfahren gemäß Anspruch 8, dadurch gekennzeichnet, daß bei den Verbindungen der allgemeinen Formeln II -IV R₂ eine Phosphoramidit-, eine H-phosphonat- oder eine Methylphosphonat-Gruppe, R₁ eine Schutzgruppe darstellt und die Kondensation durch chemische Synthese erfolgt.

11. Verwendung von Verbindungen nach den Ansprüchen 1 bis 4 zur Herstellung von Arzneimitteln mit antiviraler Wirksamkeit.

## Claims

1. Oligonucleotides which contain one or several of the heterocyclic bases mentioned in compounds of the general formulae II - IV 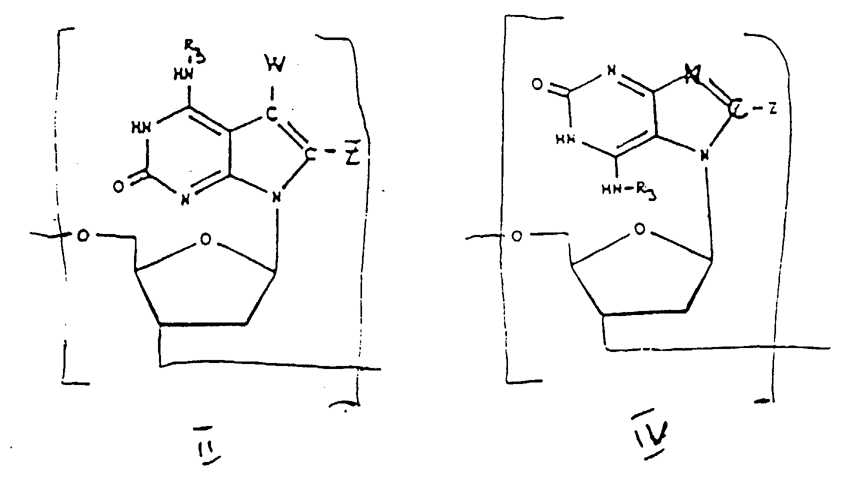 in which
R₃ = hydrogen or a protecing group,
W and Z can independently of one another denote = hydrogen, halogen, NH-(CH₂)ₙNH₂ (n = 2-12), R-CH₂COOH (R = alkylene-C 1-8), a reporter or intercalator group
and 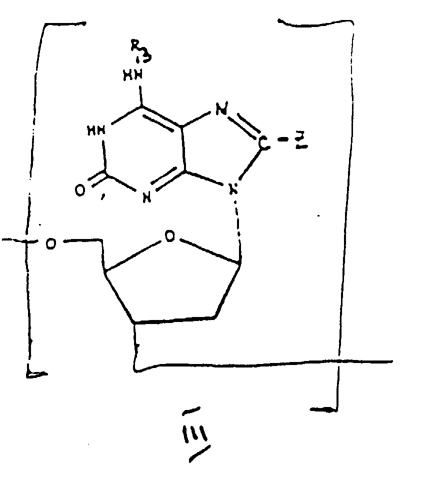 in which
R3 = hydrogen or a protecting group,
Z denotes hydrogen, halogen, NH-(CH₂)ₙNH₂ (n = 2-12), R-CH₂COOH (R = alkylene-C 1-8), a reporter or intercalator group
and are capable of forming parallel nucleic acid complexes.

2. Oligonucleotides as claimed in claim 1 which can form complementary double strands with a second oligonucleotide containing an isocytidine.

3. Oligonucleotides as claimed in claims 1 and 2, whose internucleotide bond contains methylphosphonate and/or phosphothioate groups.

4. Oligonucleotide as claimed in claims 1 to 3, wherein it is composed of 6 to 100 nucleotide building blocks.

5. Compounds of the general formula II and IV 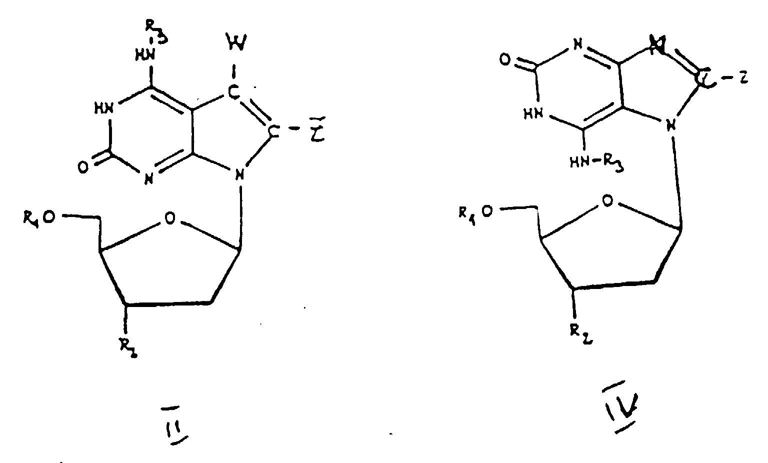 in which
R₁ = hydrogen or a protecting group, PO₃H₂, P₂O₆H₃, P₃O₉H₄ or the corresponding alpha, beta and gamma thiophosphates
R₂ = hydroxy, phosphoramidite, methylphosphonate, H-phosphonate, a reporter or intercalator group provided that R₂ is not OH if R₁, R₃, W and Z each represent hydrogen
R₃ = hydrogen or a protecting group
W and Z can independently of one another denote hydrogen, halogen, NH-(CH₂)ₙNH₂ (n = 2-12), R-CH₂COOH (R = alkylene-C 1-8),
a reporter group or intercalator group.

6. Compounds of the general formula III, 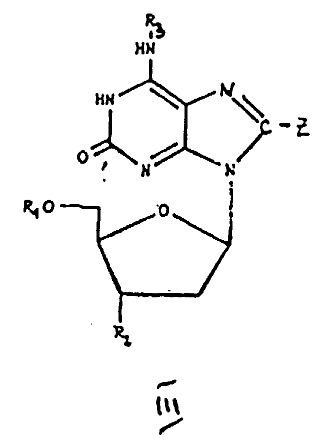 in which
R₁ = is a protecting group,
R₂ = phosphoramidite, H-phosphonate, methylphosphonate, a reporter or intercalator group
R₃ = hydrogen or a protecting group
W and Z can independently of one another denote hydrogen, halogen, NH-(CH₂)ₙNH₂ (n = 2-12), R-CH₂COOH (R = alkylene-C 1-8),
a reporter group or intercalator group.

7. Compounds as claimed in claims 5 and 6 in which the heterocyclic bases carry protecting groups.

8. Process for the production of oligonucleotides as claimed in claims 1 - 4, wherein the condensation is carried out with compounds as claimed in claims 5 - 7.

9. Process as claimed in claim 8, wherein in the compounds of the general formulae II - IV R₁ represents a triphosphate or alpha, beta or gamma thiophosphate analogues of these phosphoric acid esters and R₂ represents a reporter group or an intercalator group and the condensation is carried out enzymatically by polymerases by the "nick translation" and "random priming" methods.

10. Process as claimed in claim 8, wherein in the compounds of the general formulae II - IV R₂ represents a phosphoramidite, a H-phosphonate or a methylphosphonate group, R₁ represents a protecting group and the condensation is carried out by chemical synthesis.

11. Use of compounds as claimed in claims 10 to 13 for the production of pharmaceutical preparations with antiviral activity.

## Revendications

1. Oligonucléotides, qui contiennent une ou plusieurs des bases hétérocycliques contenues dans les composés de formules générales II-IV 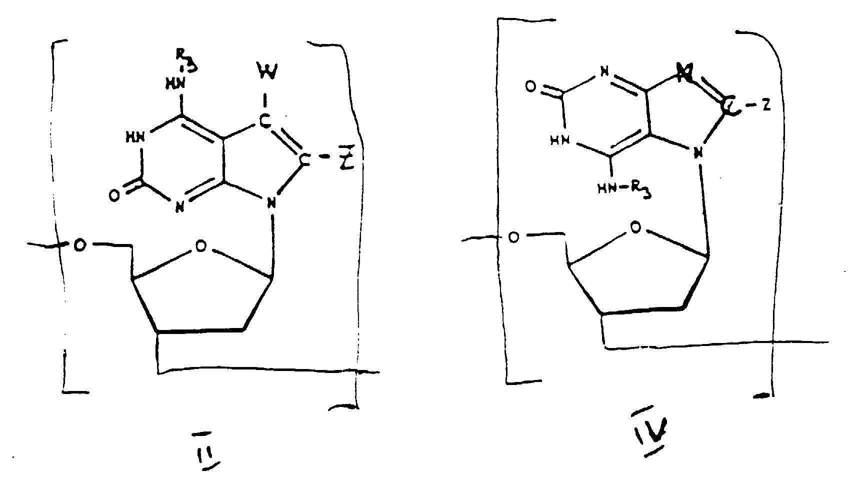 dans lesquelles
R₃ = un atome d'hydrogène ou un groupe protecteur,
W et Z peuvent représenter, indépendamment l'un de l'autre, un atome d'hydrogène, un atome d'halogène, un groupe NH-(CH₂)ₙNH₂ (n = 2-12), R-CH₂COOH (R = un groupe alkylène en ^{C}1-8^{),}
un groupe reporter ou un groupe intercalateur, et 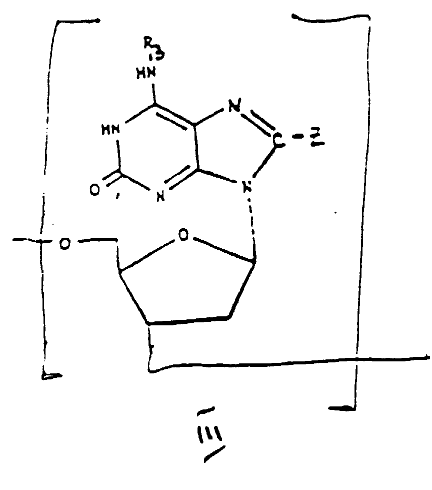 dans laquelle
R₃ = un atome d'hydrogène ou un groupe protecteur,
Z représente un atome d'hydrogène, un atome d'halogène, un groupe NH-(CH₂)ₙNH₂ (n = 2-12), R-CH₂COOH (R = un groupe alkylène en C₁₋₈),
un groupe reporter ou un groupe intercalateur,
et qui sont appropriés pour la formation de complexes d'acides nucléiques parallèles.

2. Oligonucléotides selon la revendication 1, qui peuvent former des doubles brins complémentaires avec un second oligonucléotide contenant de l'isocytidine.

3. Oligonucléotides selon les revendications 1 et 2, dont les liaisons internucléotidiques contiennent des groupes méthylphosphonate et/ou phosphothioate.

4. Oligonucléotides selon les revendications 1 à 3, caractérisés par le fait qu'ils sont constitués par 6 à 100 motifs de base nucléotidiques.

5. Composés de formules générales II et IV 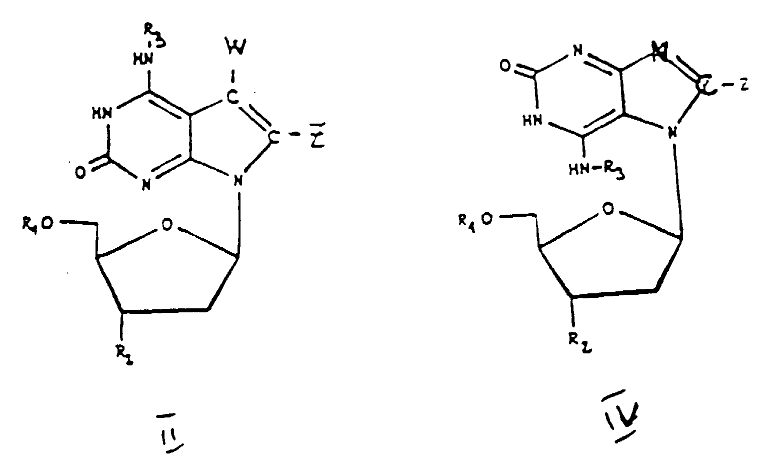 dans lesquelles
R₁ = un atome d'hydrogène ou un groupe protecteur, un groupe PO₃H₂, P₂O₆H₃, P₃O₉H₄, ou l'alpha-, béta- ou gamma-thiophosphate correspondant,
R₂ = un groupe hydroxy, phosphoramidite, méthylphosphonate, H-phosphonate, un groupe reporter ou un groupe intercalateur, étant entendu que R₂ n'est pas OH lorsque R₁, R₃, W et Z représentent chacun un atome d'hydrogène,
R₃ = un atome d'hydrogène ou un groupe protecteur,
W et Z peuvent représenter, indépendamment l'un de l'autre, un atome d'hydrogène, un atome d'halogène, un groupe NH-(CH₂)ₙNH₂ (n = 2-12), R-CH₂COOH (R = un groupe alkylène en C₁₋₈),
un groupe reporter ou un groupe intercalateur.

6. Composés de formule générale III 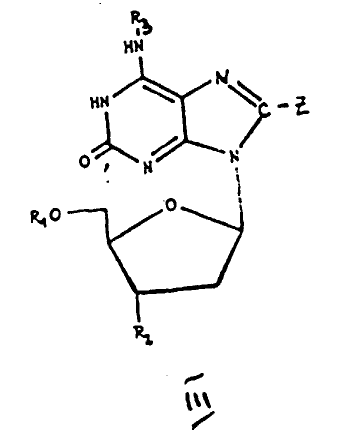 dans laquelle
R₁ = un groupe protecteur,
R₂ = un groupe phosphoramidite, H-phosphonate, méthylphosphonate, un groupe reporter ou une groupe intercalateur,
R₃ = un atome d'hydrogène ou un groupe protecteur,
W et Z peuvent représenter, indépendamment l'un de l'autre, un atome d'hydrogène, un atome d'halogène, un groupe NH-(CH₂)ₙNH₂ (n = 2-12), R-CH₂COOH (R = un groupe alkylène en C₁₋₈),
un groupe reporter ou un groupe intercalateur.

7. Composés selon les revendications 5 et 6, dans lesquels les bases hétérocycliques portent des groupes protecteurs.

8. Procédé de préparation des oligonucléotides selon les revendications 1-4, caractérisé par le fait que la condensation est réalisée avec des composés selon les revendications 5-7.

9. Procédé selon la revendication 8, caractérisé par le fait que dans les composés de formules générales II-IV, R₁ représente un triphosphate ou l'analogue α-, béta- ou gamma-thiophosphate de cet ester d'acide phosphorique, et R₂ représente un groupe reporter ou un groupe intercalateur, et que la condensation est réalisée par voie enzymatique, à l'aide de polymérases, selon les procédés par translation de coupure ("nick translation") et d'amorçage aléatoire ("random priming").

10. Procédé selon la revendication 8, caractérisé par le fait que dans les composés de formules générales II-IV, R₂ représente un groupe phosphoramidite, H-phosphonate ou méthylphosphonate, R₁ représente un groupe protecteur et que la condensation est réalisée par synthèse chimique.

11. Utilisation de composés selon les revendications 1 à 4, pour la préparation de médicaments ayant une activité antivirale.
